# EUROPEAN PATENT APPLICATION

(11) **EP 4 023 400 A1**
(43) Date of publication of application: **06.07.2022**
(21) Application number: 20856481.5
(22) Date of filing: 28.08.2020
(51) Int. Cl.: B25J 17/00, F16H 3/089, A61F 2/60

(54) **JOINT DEVICE**

(30) Priority: 29.08.2019 JP 2019157358
(71) Applicant: HONDA MOTOR CO., LTD., Minato-ku Tokyo 107-8556 (JP)
(72) Inventor: SHIMADA Kei, Wako-shi, Saitama 351-0193 (JP)
(74) Representative: Weickmann & Weickmann PartmbB
(86) International application number: PCT/JP2020/032787
(87) International publication number: WO 2021/040039

(57) **Abstract**

An electric prosthetic limb 1 is provided with: a below-knee side member 10; an above-knee side member 20; a knee joint mechanism 30 that connects the below-knee side member 10 and the above-knee side member 20 in a manner such that an angle formed therebetween is variable; and an extendable device 40 that is capable of extending and contracting to change the angle formed between the below-knee side member 10 and the above-knee side member 20. The extendable device 40 is provided with a motor M and a transmission T transmitting power from the motor M. The transmission T is provided with: a first transmission mechanism T1 that transmits the power from the motor M at a first transmission gear ratio; and a second transmission mechanism T2 that transmits the power from the motor M at a second transmission gear ratio different from the first transmission gear ratio.

## Description

### TECHNICAL FIELD

The present invention relates to a joint device.

### BACKGROUND ART

In the related art, as a joint device used in a connecting unit that connects two members, there is a joint device including an expansion and contraction device capable of changing an angle formed by the two members. As such a joint device, for example, there is a prosthetic leg used for a knee joint. Patent Literature 1 discloses that a sensor for detecting a contraction motion of muscles at a cut end portion of a cut leg is provided in a thigh socket of a prosthetic leg attached to the cut end portion of the cut leg, and a throttle degree of a variable valve of a hydraulic cylinder for adjusting resistance to bending and extension of a knee joint portion is controlled based on detection information from the sensor.

Patent Literature 1: JP H11-19105 A

However, the prosthetic leg disclosed in Patent Literature 1 can generate the resistance to bending and extension, but cannot generate power for bending and extension. In particular, in order to go up the stairs smoothly, it is necessary to extend the knee joint while a load is applied.

The present invention provides a joint device capable of extending and bending a connecting unit by power of a power source.

### SUMMARY OF INVENTION

There is provided a joint device that includes:
a first member;
a second member;
a connecting unit connecting the first member and the second member such that an angle formed by the first member and the second member is variable; and
an expansion and contraction device capable of varying the angle formed by the first member and the second member by expansion and contraction, in which
the expansion and contraction device includes:
   a power source; and
   a power transmission unit configured to transmit power of the power source, and
the power transmission unit includes:
   a first power transmission path through which the power is transmitted at a first transmission gear ratio; and
   a second power transmission path through which the power is transmitted at a second transmission gear ratio different from the first transmission gear ratio.

According to the present invention, the connecting unit can be extended and bent via the power transmission unit that transmits the power of the power source.

### BRIEF DESCRIPTION OF DRAWINGS

Fig. 1 is a perspective view of an electric prosthetic leg according to a first embodiment of the present invention as viewed obliquely from a front.
Fig. 2 is a perspective view of the electric prosthetic leg of Fig. 1 as viewed obliquely from a rear.
Fig. 3 is a view showing an internal structure of the electric prosthetic leg of Fig. 1.
Fig. 4 is an enlarged view of a power transmission unit of the electric prosthetic leg of Fig. 1.
Fig. 5A is a view showing power transmission of the power transmission unit when the power transmission unit of Fig. 4 is in a first speed change state.
Fig. 5B is a view showing power transmission of the power transmission unit when the power transmission unit of Fig. 4 is in a second speed change state.
Fig. 5C is a view showing power transmission of the power transmission unit when the power transmission unit of Fig. 4 is in a free state.
Fig. 6 is a diagram showing power for extending a knee joint mechanism from a bent state when going up stairs.
Fig. 7 is a diagram showing power for bending the knee joint mechanism from an extended state when going up the stairs.
Fig. 8 is a perspective view of a power transmission unit of an electric prosthetic leg according to a second embodiment of the present invention.
Fig. 9 is a cross-sectional view of the power transmission unit of Fig. 8.

### DESCRIPTION OF EMBODIMENTS

Hereinafter, embodiments of an electric prosthetic leg as an example of a joint device of the present invention will be described with reference to the drawings. In the following description, a front-rear direction, a left-right direction, and an up-down direction are defined with reference to the user of the electric prosthetic leg. In the drawings, the front, rear, left, right, upper, and lower sides of the electric prosthetic leg are represented by Fr, Rr, L, R, U, and D, respectively.

### <First Embodiment>

### [Electric Prosthetic Leg]

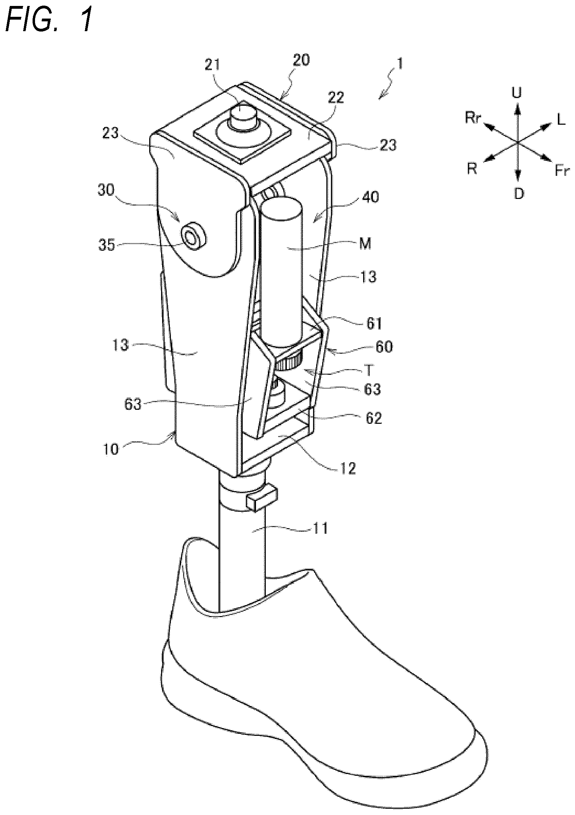

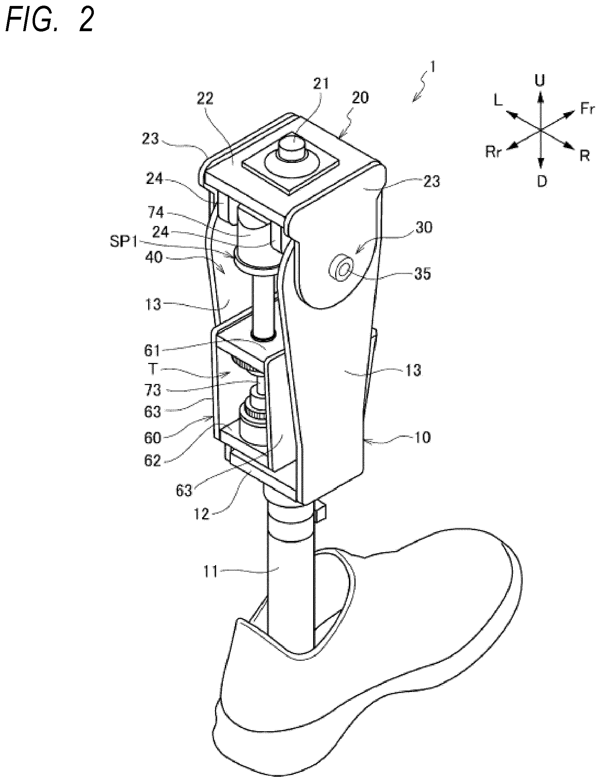

As shown in Figs. 1 to 3, an electric prosthetic leg 1 according to the present embodiment is a prosthetic leg for a person who does not have a knee, and includes a knee lower side member 10 below the knee, a knee upper side member 20 above the knee, a knee joint mechanism 30 that connects the knee lower side member 10 and the knee upper side member 20 such that an angle formed by the knee lower side member 10 and the knee upper side member 20 is variable, and an expansion and contraction device 40 that can vary the angle formed by the knee lower side member 10 and the knee upper side member 20 by expansion and contraction.

The knee upper side member 20 includes an upper wall portion 22 provided with an adapter 21 connected to a socket (not shown), and a pair of upper side wall portions 23 extending downward from both left and right ends of the upper wall portion 22, and has a substantially U shape that opens downward when viewed from the front-rear direction.

The knee lower side member 10 includes a lower wall portion 12 provided with a foot portion 11, and a pair of lower side wall portions 13 extending upward from both left and right ends of the lower wall portion 12, and has a substantially U shape that opens upward when viewed from the front-rear direction.

The pair of lower side wall portions 13 of the knee lower side member 10 are connected between the pair of upper side wall portions 23 of the knee upper side member 20 so as to be rotatable about rotating portions 35. With this mechanism, the knee joint mechanism 30 is formed by connecting the knee lower side member 10 and the knee upper side member 20 such that the angle formed by the knee lower side member 10 and the knee upper side member 20 is variable.

In a space formed between the knee upper side member 20 and the knee lower side member 10, the expansion and contraction device 40 capable of varying the angle formed by the knee lower side member 10 and the knee upper side member 20 is provided.

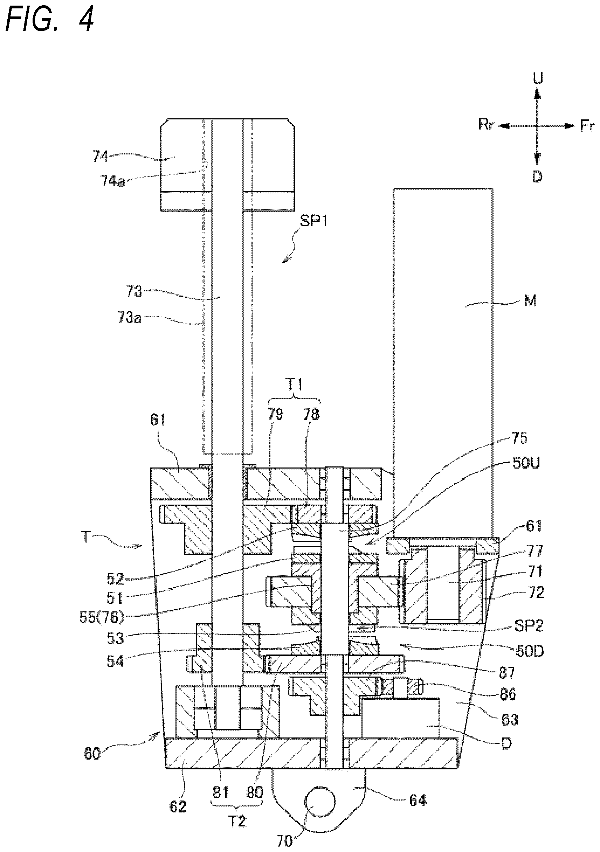

Referring also to Fig. 4, the expansion and contraction device 40 includes a motor M that outputs rotary power, a transmission T that transmits the power of the motor M, a first spindle unit SP1 that is connected to the transmission T so as to transmit the power and converts the rotary power output from the transmission T into translational motion, an connection and disconnection mechanism 50 interposed between the motor M and a first transmission mechanism T1 and a second transmission mechanism T2 of the transmission T to be described later, and a second spindle unit SP2 that converts the rotary power output from the motor M into translational motion of an actuator 55 of the connection and disconnection mechanism 50.

The transmission T includes a transmission case 60 including a top plate portion 61, a bottom plate portion 62, and a pair of side plate portions 63 that connect both left and right ends of the top plate portion 61 and the bottom plate portion 62, and having a rectangular shape when viewed in the front-rear direction. In the transmission case 60, a pair of rotary blades 64 extending downward from the bottom plate portion 62 are supported by a rotary support wall 14 extending upward from the lower wall portion 12 of the knee lower side member 10 so as to be swingable and immovable about a lower swing portion 70.

The motor M is disposed in front of and above the top plate portion 61 of the transmission case 60 such that an output shaft 71 of the motor M passes through the top plate portion 61 and protrudes into the transmission case 60. The first spindle unit SP1 is disposed on a side opposite to the motor M with the connection and disconnection mechanism 50 sandwiched therebetween in the front-rear direction. In other words, the motor M is disposed forward of the connection and disconnection mechanism 50 in the front-rear direction, and the first spindle unit SP1 is disposed rearward of the connection and disconnection mechanism 50 in the front-rear direction. Accordingly, even when the motor M is mounted on the electric prosthetic leg 1, the electric prosthetic leg 1 can be prevented from becoming large in a width direction while maintaining a balance in the front-rear direction.

The second spindle unit SP2 that converts the rotary power output from the motor M into the translational motion of the actuator 55 of the connection and disconnection mechanism 50 is disposed between the motor M and the first spindle unit SP1 in the front-rear direction. The output shaft 71 of the motor M, a first spindle 73 of the first spindle unit SP1, and a second spindle 75 of the second spindle unit SP2 are disposed parallel to each other, and are disposed to extend in an up-down direction in a state in which the knee joint mechanism 30 is completely extended.

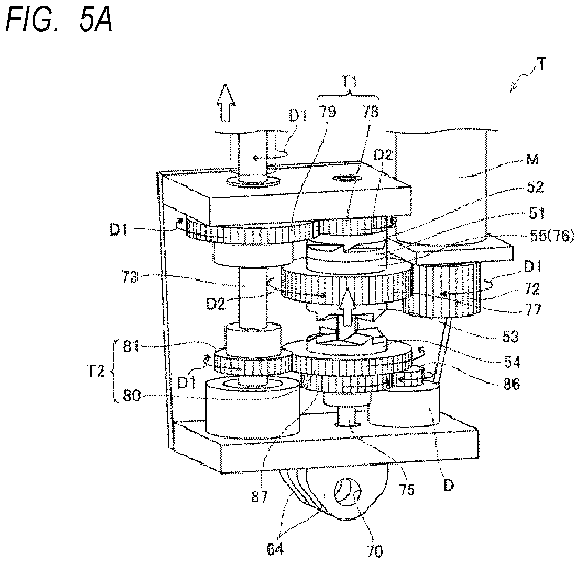

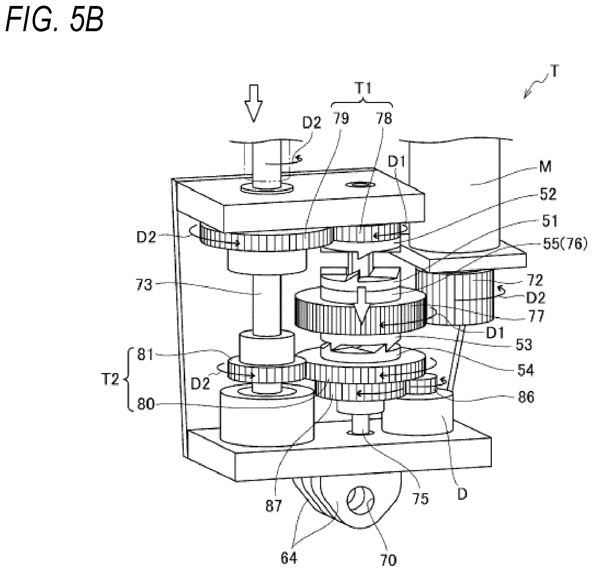

The first spindle unit SP1 includes the first spindle 73 in which a male screw 73a is formed and a sleeve 74 in which a female screw 74a is formed, and the sleeve 74 performs the translational motion along an axial center of the first spindle 73 by rotation of the first spindle 73. In the present embodiment, the first spindle 73 receives the rotary power of the motor M transmitted by the transmission T to perform rotational motion. Meanwhile, the sleeve 74 is attached to a pair of inner side wall portions 24 extending downward from the upper wall portion 22 of the knee upper side member 20 so as to be swingable and immovable about an upper swing portion 25. Therefore, when the first spindle 73 receives the rotary power of the motor M transmitted by the transmission T and rotates to one side (a direction of an arrow D1 in Fig. 5A), the sleeve 74 moves in a translational manner so as to separate from the transmission T, and when the first spindle 73 rotates to the other side (a direction of an arrow D2 in Fig. 5B), the sleeve 74 moves in the translational manner so as to approach the transmission T.

That is, a distance between the sleeve 74 and the transmission T increases or decreases in accordance with a rotation direction of the first spindle 73. Since the sleeve 74 is immovably attached to the knee upper side member 20 as described above, the knee lower side member 10 to which the transmission T is attached and the knee upper side member 20 to which the sleeve 74 is attached rotate about the rotating portions 35 as the distance between the sleeve 74 and the transmission T increasing or decreasing in accordance with the rotation direction of the first spindle 73. Accordingly, the angle formed by the knee upper side member 20 and the knee lower side member 10 changes.

As shown in Fig. 4, the transmission T includes an output gear 72 provided on the output shaft 71 of the motor M, an input gear 77 that is provided at a substantially central portion of the second spindle 75 of the second spindle unit SP2 and meshes with the output gear 72, the first transmission mechanism T1, and the second transmission mechanism T2.

The first transmission mechanism T1 includes a first drive gear 78 that is immovably provided on an upper portion of the second spindle 75 of the second spindle unit SP2, and a first driven gear 79 that is provided on the first spindle 73 of the first spindle unit SP1 so as to rotate integrally with the first spindle 73 and meshes with the first drive gear 78.

The second transmission mechanism T2 includes a second drive gear 80 that is immovably provided on a lower portion of the second spindle 75 of the second spindle unit SP2, and a second driven gear 81 that is provided on the first spindle 73 of the first spindle unit SP1 so as to rotate integrally with the first spindle 73 and meshes with the second drive gear 80.

The first transmission mechanism T1 transmits the power of the motor M at a first transmission gear ratio. The second transmission mechanism T2 transmits the power of the motor M at a second transmission gear ratio different from the first transmission gear ratio. By providing the two power transmission paths having different transmission gear ratios, operation speeds and generation power of extension and bending in the knee joint mechanism 30 can be switched. It is sufficient that the first transmission gear ratio and the second transmission gear ratio are different from each other, and either one of the first transmission mechanism T1 and the second transmission mechanism T2 may be a speed reduction mechanism and the other may be a speed increase mechanism, either one may be a constant speed mechanism and the other may be the speed reduction mechanism or the speed increase mechanism, both may be the speed reduction mechanisms, or both may be the speed increase mechanisms.

A speed reduction ratio of the first transmission gear ratio is preferably larger than that of the second transmission gear ratio. Accordingly, the first drive gear 78 has a smaller diameter than that of the second drive gear 80, and the first drive gear 78 and the motor M can be disposed close to each other. In the present embodiment, the first transmission mechanism T1 is disposed closer to the motor M than the second transmission mechanism T2. More specifically, the motor M is disposed in front of the first drive gear 78 so as to overlap the first drive gear 78 in the up-down direction.

The first transmission mechanism T1 and the second transmission mechanism T2 are switched by the connection and disconnection mechanism 50. The connection and disconnection mechanism 50 includes an upper clutch 50U interposed between the motor M and the first transmission mechanism T1, a lower clutch 50D interposed between the motor M and the second transmission mechanism T2, and the actuator 55 that rotates integrally with the input gear 77.

The upper clutch 50U is a dog clutch including a first engagement element 51 that is an engagement element on a motor M side, and a second engagement element 52 that is an engagement element on a first transmission mechanism T1 side. More specifically, the first engagement element 51 is provided over the actuator 55 and the input gear 77 so as to rotate integrally with the input gear 77. The second engagement element 52 is provided under the first drive gear 78 so as to rotate integrally with the first drive gear 78 and so as to be engageable with the first engagement element 51. The second engagement element 52 and the first drive gear 78 are mounted on the upper portion of the second spindle 75 of the second spindle unit SP2 so as to be rotatable and immovable relative to the second spindle 75.

The lower clutch 50D is a dog clutch including a third engagement element 53 that is an engagement element on the motor M side, and a fourth engagement element 54 that is an engagement element on a second transmission mechanism T2 side. More specifically, the third engagement element 53 is provided under the actuator 55 and the input gear 77 so as to rotate integrally with the input gear 77. The fourth engagement element 54 is provided over the second drive gear 80 so as to rotate integrally with the second drive gear 80 and so as to be engageable with the third engagement element 53. The fourth engagement element 54 and the second drive gear 80 are mounted on a lower portion of the second spindle 75 of the second spindle unit SP2 so as to be rotatable and immovable relative to the second spindle 75.

The actuator 55 is attached to the input gear 77 so as to rotate integrally with the input gear 77 as described above, and the first engagement element 51 and the third engagement element 53 are attached to an upper portion and a lower portion of the actuator 55 so as to rotate integrally. The actuator 55 is interposed substantially at a center of the second spindle 75, that is, between the first drive gear 78 and the second drive gear 80 in the up-down direction. Here, the actuator 55 is a screw nut 76 of the second spindle unit SP2. The second spindle unit SP2 includes the second spindle 75 in which a male screw is formed and the screw nut 76 (actuator 55) in which a female screw is formed, and the screw nut 76 (actuator 55) performs translational motion while rotating along an axial center of the second spindle 75 with rotation of the input gear 77.

The screw nut 76 (the actuator 55) moves upward in the up-down direction, which is a moving direction of the translational motion, when the motor M rotates in the first direction (the direction of the arrow D1 in Fig. 5A), and moves downward in the up-down direction when the motor M rotates in a second direction (the direction of the arrow D2 in Fig. 5B) opposite to the first direction. In this way, by changing a rotation direction of the motor M, a moving direction of the actuator 55 can be changed.

The connection and disconnection mechanism 50 can have three states including a first speed change state, a second speed change state, and a free state by the actuator 55 performing the translational motion along the axial center of the second spindle 75.

In the first speed change state, as shown in Fig. 5A, the motor M rotates in the first direction (the direction of the arrow D1 in Fig. 5A) and the screw nut 76 (the actuator 55) moves upward, and thereby the first engagement element 51 and the second engagement element 52 are connected with each other and the third engagement element 53 and the fourth engagement element 54 are disconnected from each other. In other words, the upper clutch 50U is engaged, and the lower clutch 50D is released. In the first speed change state, the power of the motor M is transmitted to the output gear 72, the input gear 77, the actuator 55, the first engagement element 51, the second engagement element 52, the first drive gear 78, the first driven gear 79, and the first spindle unit SP1.

In the second speed change state, as shown in Fig. 5B, the motor M rotates in the second direction (the direction of the arrow D2 in Fig. 5B) and the screw nut 76 (the actuator 55) moves downward, and thereby the first engagement element 51 and the second engagement element 52 are disconnected from each other and the third engagement element 53 and the fourth engagement element 54 are connected with each other. In other words, the upper clutch 50U is released, and the lower clutch 50D is engaged. In the second speed change state, the power of the motor M is transmitted to the output gear 72, the input gear 77, the actuator 55, the third engagement element 53, the fourth engagement element 54, the second drive gear 80, the second driven gear 81, and the first spindle unit SP1.

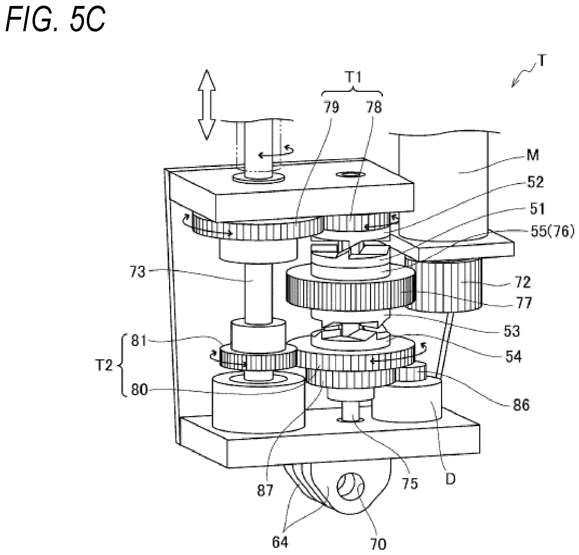

In the free state, as shown in Fig. 5C, the first engagement element 51 and the second engagement element 52 are disconnected from each other, and the third engagement element 53 and the fourth engagement element 54 are disconnected from each other. In other words, the upper clutch 50U is released, and the lower clutch 50D is released. In the free state, the motor M is stopped, and the first driven gear 79 and the second driven gear 81 are rotated due to the rotation of the first spindle unit SP1. The rotation of the first spindle unit SP1 is transmitted to the first driven gear 79, the first drive gear 78, and the second engagement element 52, but is not transmitted to the first engagement element 51. Similarly, the rotation of the first spindle unit SP1 is transmitted to the second driven gear 81, the second drive gear 80, and the fourth engagement element 54, but is not transmitted to the third engagement element 53.

In Figs. 4 to 5C, a reference numeral D indicates a rotary damper that applies an appropriate resistance to the second spindle 75 of the second spindle unit SP2 such that the screw nut 76 (the actuator 55) can reliably move in the translational manner when the motor M is rotated. The rotary damper D includes a first damper gear 86 provided on a rotary shaft of the rotary damper D, and a second damper gear 87 provided on the second spindle 75 of the second spindle unit SP2 so as to rotate integrally with the second spindle 75 and meshing with the first damper gear 86. The rotary damper D is disposed on a front portion of the bottom plate portion 62 of the transmission case 60 and below the motor M.

The electric prosthetic leg 1 configured as described above can smoothly perform a going-up-stairs motion, which requires going up one stair per time on a non-prosthetic side leg with a passive prosthetic leg including a passive damper in the related art.

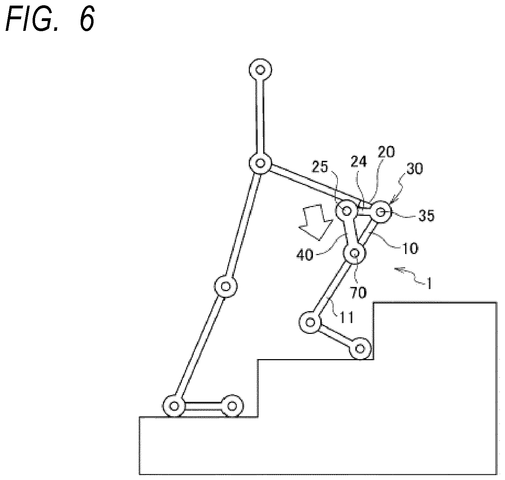

Specifically, as shown in Fig. 6, when the electric prosthetic leg 1 is pushed forward to go up the stairs, a large power is necessary to extend the knee joint mechanism 30 from a bent state in a state in which a load is applied to the electric prosthetic leg 1.

At this time, as shown in Fig. 5A, by rotating the motor M in the first direction (the direction of the arrow D1 in Fig. 5A), the power of the motor M is transmitted from the output gear 72 to the input gear 77. When the input gear 77 rotates in the second direction (the direction of the arrow D2 in Fig. 5A), the actuator 55 moves upward being guided by the second spindle 75 while rotating around the second spindle 75 of the second spindle unit SP2. Then, the first engagement element 51 provided over the actuator 55 and the input gear 77 engages with the second engagement element 52 provided under the first drive gear 78, and the connection and disconnection mechanism 50 is in the first speed change state.

When the connection and disconnection mechanism 50 is in the first speed change state, the power of the motor M is transmitted to the output gear 72, the input gear 77, the actuator 55, the first engagement element 51, the second engagement element 52, the first drive gear 78, the first driven gear 79, and the first spindle unit SP1. When the first drive gear 78 rotates in the second direction (the direction of the arrow D2 in Fig. 5A) together with the input gear 77, the first driven gear 79 rotates in the first direction (the direction of the arrow D1 in Fig. 5A), and when the first driven gear 79 rotates in the first direction (the direction of the arrow D1 in Fig. 5A), the first spindle 73 of the first spindle unit SP1 rotates in the first direction (the direction of the arrow D1 in Fig. 5A). Accordingly, the sleeve 74 moves in the translational manner so as to move away from the transmission T, and the knee upper side member 20 to which the sleeve 74 is attached rotates about the rotating portions 35 with respect to the knee lower side member 10 to which the transmission T is attached, and thereby the knee joint mechanism 30 extends.

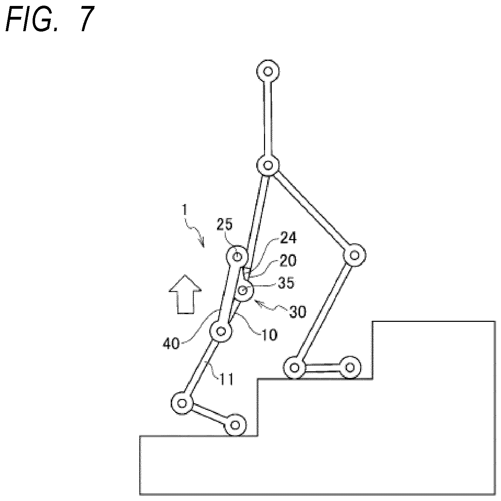

Meanwhile, in order to smoothly perform the going-up-stairs motion, as shown in Fig. 7, it is necessary to bend (lift) the knee joint mechanism 30 from an extended state in a state in which a load is applied to a healthy leg. When the knee joint mechanism 30 is bent from the extended state, large power is not required, but a quick operation is required.

At this time, as shown in Fig. 5B, by rotating the motor M in the second direction (the direction of the arrow D2 in Fig. 5B), the power of the motor M is transmitted from the output gear 72 to the input gear 77. When the input gear 77 rotates in the first direction (the direction of the arrow D1 in Fig. 5B), the actuator 55 moves downward being guided by the second spindle 75 while rotating around the second spindle 75 of the second spindle unit SP2. Then, the third engagement element 53 provided under the actuator 55 and the input gear 77 engages with the fourth engagement element 54 provided over the second drive gear 80, and the connection and disconnection mechanism 50 is in the second speed change state.

When the connection and disconnection mechanism 50 is in the second speed change state, the power of the motor M is transmitted to the output gear 72, the input gear 77, the actuator 55, the third engagement element 53, the fourth engagement element 54, the second drive gear 80, the second driven gear 81, and the first spindle unit SP1. When the second drive gear 80 rotates in the first direction (the direction of the arrow D1 in Fig. 5B) together with the input gear 77, the second driven gear 81 rotates in the second direction (the direction of the arrow D2 in Fig. 5B), and when the second driven gear 81 rotates in the second direction (the direction of the arrow D2 in Fig. 5B), the first spindle 73 of the first spindle unit SP1 rotates in the second direction (the direction of the arrow D2 in Fig. 5B). Accordingly, the sleeve 74 moves in the translational manner so as to approach the transmission T, the knee lower side member 10 to which the transmission T is attached rotates about the rotating portions 35 with respect to the knee upper side member 20 to which the sleeve 74 is attached, and the knee joint mechanism 30 is bent.

Further, by setting the connection and disconnection mechanism 50 to the free state during normal walking, the power transmission between the motor M and the transmission T can be cut off, which prevents walking from being hindered.

In the electric prosthetic leg 1 configured as described above, the knee joint mechanism 30 can be extended and bent via the transmission T that transmits the power of the motor M. A rotation range of the knee joint mechanism 30 is limited to 180°or less, the angle formed by the knee lower side member 10 and the knee upper side member 20 is approximately 180° when the knee joint mechanism 30 is in the extended state, and the angle formed by the knee lower side member 10 and the knee upper side member 20 is less than 180° when the knee joint mechanism 30 is in the bent state.

Since the transmission T includes the two power transmission paths having different transmission gear ratios, the operation speeds and the generation power of the extension and bending in the knee joint mechanism 30 can be switched. In particular, the required operation speeds and the generation power are different when the knee joint mechanism 30 is bent and extended when going up the stairs, and thus the power transmission paths can be changed when the knee joint mechanism 30 is bent and extended.

Further, since the connection and disconnection mechanism 50 interposed between the motor M and the transmission T includes the upper clutch 50U interposed between the motor M and the first transmission mechanism T1 and the lower clutch 50D interposed between the motor M and the second transmission mechanism T2, the two power transmission paths can be appropriately switched.

In particular, since the expansion and contraction device 40 includes the second spindle unit SP2 that converts the rotary power output from the motor M into the translational motion of the actuator 55, control of the actuator 55 and control of the extension and bending of the knee joint mechanism 30 can be implemented by one motor M. Further, since the transmission T is configured to be switchable among the first speed change state, the second speed change state, and the free state by the single actuator 55, the two power transmission paths can be prevented from being connected at the same time.

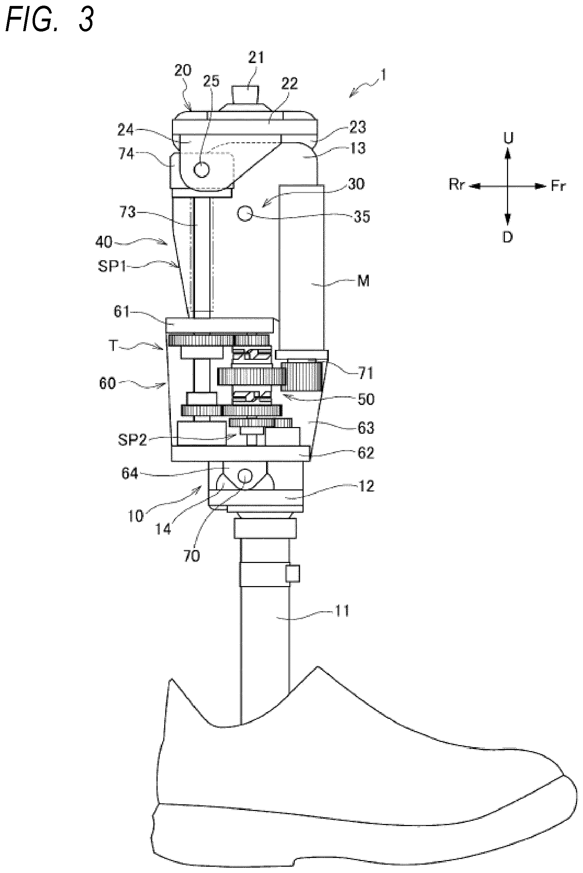

When the electric prosthetic leg 1 in which the knee joint mechanism 30 is in the extended state is viewed along a rotation axis of the rotating portions 35 of the knee joint mechanism 30, a region in which the angle formed by the knee lower side member 10 and the knee upper side member 20 is less than 180° (a region on a rear (calf) side of a line connecting the rotating portion 35 and the lower swing portion 70 in Fig. 3) is referred to as a narrow angle region, a region in which the angle formed by the knee lower side member 10 and the knee upper side member 20 is 180° or more (a region on a front (tibial) side of the line connecting the rotating portion 35 and the lower swing portion 70 in Fig. 3) is referred to as a wide angle region, and the first spindle unit SP1 is disposed in the narrow angle region. Meanwhile, the motor M is disposed in the wide angle region. By disposing the first spindle unit SP1 in the narrow angle region and disposing the motor M in the wide angle region in this manner, the first spindle unit SP1 and the motor M can be disposed in a balanced manner with the rotating portions 35 of the knee joint mechanism 30 sandwiched therebetween. Further, the transmission T and the connection and disconnection mechanism 50 are disposed between the motor M and the first spindle unit SP1. Therefore, the motor M, the transmission T, the connection and disconnection mechanism 50, and the first spindle unit SP1 can be collectively disposed.

### <Second Embodiment>

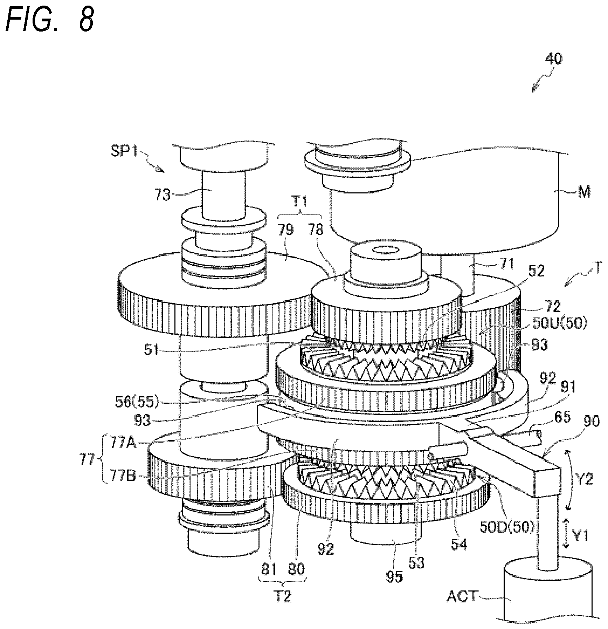

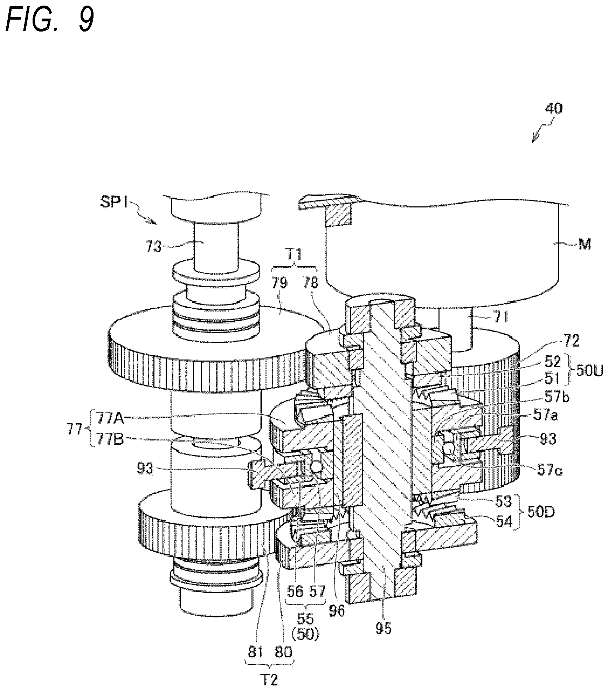

The electric prosthetic leg 1 according to a second embodiment has a different configuration of the expansion and contraction device 40 from that according to the first embodiment. More specifically, in the expansion and contraction device 40 according to the first embodiment, a translational motion of the actuator 55 is implemented by converting rotary power output from the motor M by the second spindle unit SP2. In contrast, in the expansion and contraction device 40 according to the second embodiment, a translational motion of the actuator 55 is implemented by a clutch actuator ACT serving as a drive source different from the motor M, and a clutch fork 90 that transmits power of the clutch actuator ACT to the actuator 55. In the following description, the expansion and contraction device 40 of the electric prosthetic leg 1 according to the second embodiment will be described with reference to Figs. 8 and 9. The same or equivalent configurations as those of the expansion and contraction device 40 of the electric prosthetic leg 1 according to the first embodiment are denoted by the same reference numerals in the drawings and descriptions thereof will be omitted, and only differences will be described.

As shown in Figs. 8 and 9, the expansion and contraction device 40 according to the second embodiment includes the motor M that outputs the rotary power, the transmission T that transmits power of the motor M, the first spindle unit SP1 that is connected to the transmission T to transmit the power and converts the rotary power output from the transmission T into the translational motion, the connection and disconnection mechanism 50 interposed between the motor M and the first transmission mechanism T1 and the second transmission mechanism T2 of the transmission T, a support shaft 95 that is disposed parallel to the output shaft 71 of the motor M and the first spindle 73 of the first spindle unit SP1 and supports the connection and disconnection mechanism 50, the clutch actuator ACT that performs the translational motion, and the clutch fork 90 that transmits the power of the clutch actuator ACT to the actuator 55 of the connection and disconnection mechanism 50.

The clutch actuator ACT is a power source different from the motor M, and performs the translational motion along an axial center direction (up-down direction) of the support shaft 95 as indicated by an arrow Y1 in Fig. 8.

One end portion of the clutch fork 90 is connected to the clutch actuator ACT, and an intermediate portion of the clutch fork 90 is supported by a swing shaft 65, and thereby the clutch fork 90 is swingable about the swing shaft 65 as indicated by an arrow Y2 in Fig. 8. The other end portion of the clutch fork 90 is provided with arms 92 that are two parts branching from a branch portion 91 located on a side opposite to the clutch actuator ACT with respect to the swing shaft 65 and extend in opposite directions in an arc shape. A connecting pin 93 fitted to a slide clutch 56 to be described later is provided at a distal end portion of each of the arms 92. Therefore, when the clutch actuator ACT performs the translational motion, the clutch fork 90 swings about the swing shaft 65, and the connecting pins 93 of the clutch fork 90 swing up and down.

The transmission T includes the output gear 72 provided on the output shaft 71 of the motor M, a first input gear 77A and a second input gear 77B that are provided in a substantially central portion of the support shaft 95 and mesh with the output gear 72, the first transmission mechanism T1, and the second transmission mechanism T2. The first input gear 77A and the second input gear 77B constitute the input gear 77.

The first transmission mechanism T1 includes the first drive gear 78 that is immovably provided on an upper portion of the support shaft 95, and the first driven gear 79 that is provided on the first spindle 73 of the first spindle unit SP1 so as to rotate integrally with the first spindle 73 and meshes with the first drive gear 78.

The second transmission mechanism T2 includes the second drive gear 80 that is immovably provided on a lower portion of the support shaft 95, and the second driven gear 81 that is provided on the first spindle 73 of the first spindle unit SP1 so as to rotate integrally with the first spindle 73 and meshes with the second drive gear 80.

Transmission gear ratios of the first transmission mechanism T1 and the second transmission mechanism T2 are the same as those in the first embodiment, and therefore descriptions thereof will be omitted here. The first transmission mechanism T1 and the second transmission mechanism T2 are switched by the connection and disconnection mechanism 50. The connection and disconnection mechanism 50 includes the upper clutch 50U interposed between the motor M and the first transmission mechanism T1, the lower clutch 50D interposed between the motor M and the second transmission mechanism T2, and the actuator 55.

The upper clutch 50U is a dog clutch including the first engagement element 51 that is an engagement element on a motor M side, and the second engagement element 52 that is an engagement element on a first transmission mechanism T1 side. More specifically, the first engagement element 51 is provided over the first input gear 77A so as to rotate integrally with the first input gear 77A. The second engagement element 52 is provided under the first drive gear 78 so as to rotate integrally with the first drive gear 78 and so as to be engageable with the first engagement element 51. The second engagement element 52 and the first drive gear 78 are mounted on the upper portion of the support shaft 95 so as to be rotatable and immovable relative to the support shaft 95.

The lower clutch 50D is a dog clutch including the third engagement element 53 that is an engagement element on the motor M side, and the fourth engagement element 54 that is an engagement element on a second transmission mechanism T2 side. More specifically, the third engagement element 53 is provided under the second input gear 77B so as to rotate integrally with the second input gear 77B. The fourth engagement element 54 is provided over the second drive gear 80 so as to rotate integrally with the second drive gear 80 and so as to be engageable with the third engagement element 53. The fourth engagement element 54 and the second drive gear 80 are attached on the lower portion of the support shaft 95 so as to be rotatable and immovable relative to the support shaft 95.

The actuator 55 includes the annular slide clutch 56 that is constantly engaged with the connecting pins 93 of the clutch fork 90, and a bearing 57 that performs the translational motion together with the slide clutch 56, and is disposed between the first input gear 77A and the second input gear 77B in the up-down direction.

The slide clutch 56 is provided with connecting holes with which the connecting pins 93 of the clutch fork 90 are engaged, and the connecting holes absorb swinging of the clutch fork 90 and convert the swinging into the translational motion of the slide clutch 56 in the up-down direction.

The bearing 57 includes an outer ring 57a that is non-rotatably supported by the slide clutch 56, an inner ring 57b that is configured to rotate integrally with the first input gear 77A provided with the first engagement element 51 and the second input gear 77B provided with the third engagement element 53, and a rolling element 57c that is disposed between the outer ring 57a and the inner ring 57b and allows relative rotation between the outer ring 57a and the inner ring 57b.

The inner ring 57b is supported by an outer peripheral portion of a support flange 96 key-connected to the support shaft 95 together with the first input gear 77A and the second input gear 77B so as to be translatable in the up-down direction.

The slide clutch 56 and the bearing 57 (actuator 55) move upward along the support shaft 95 when one end portion of the clutch fork 90 moves downward by the clutch actuator ACT, and move downward along the support shaft 95 when one end portion of the clutch fork 90 moves upward by the clutch actuator ACT. A predetermined gap is provided in the up-down direction between the slide clutch 56, the outer ring 57a, and the first input gear 77A, such that the first input gear 77A does not interfere with the slide clutch 56 and the outer ring 57a that are non-rotating members. Similarly, a predetermined gap is provided in the up-down direction between the slide clutch 56, the outer ring 57a, and the second input gear 77B, such that the second input gear 77B does not interfere with the slide clutch 56 and the outer ring 57a which are the non-rotating members.

The connection and disconnection mechanism 50 can have three states of a first speed change state, a second speed change state, and a free state by the slide clutch 56 and the bearing 57 (actuator 55) performing the translational motion in the up-down direction along the axial center of the support shaft 95.

In the first speed change state, as in Fig. 5A, the slide clutch 56 and the bearing 57 (the actuator 55) move upward, and thereby the first engagement element 51 and the second engagement element 52 are connected with each other and the third engagement element 53 and the fourth engagement element 54 are disconnected from each other. In other words, the upper clutch 50U is engaged, and the lower clutch 50D is released. In the first speed change state, the power of the motor M is transmitted to the output gear 72, the first input gear 77A (the inner ring 57b and the second input gear 77B), the first engagement element 51, the second engagement element 52, the first drive gear 78, the first driven gear 79, and the first spindle unit SP1.

In the second speed change state, as in Fig. 5B, the slide clutch 56 and the bearing 57 (the actuator 55) move downward, and thereby the first engagement element 51 and the second engagement element 52 are disconnected from each other and the third engagement element 53 and the fourth engagement element 54 are connected with each other. In other words, the upper clutch 50U is released, and the lower clutch 50D is engaged. In the second speed change state, the power of the motor M is transmitted to the output gear 72, the second input gear 77B (the inner ring 57b and the first input gear 77A), the third engagement element 53, the fourth engagement element 54, the second drive gear 80, the second driven gear 81, and the first spindle unit SP1.

In the free state, as in Fig. 5C, the first engagement element 51 and the second engagement element 52 are disconnected from each other, and the third engagement element 53 and the fourth engagement element 54 are disconnected from each other. In other words, the upper clutch 50U is released, and the lower clutch 50D is released. In the free state, the motor M is stopped, and the first driven gear 79 and the second driven gear 81 are rotated due to the rotation of the first spindle unit SP1. The rotation of the first spindle unit SP1 is transmitted to the first driven gear 79, the first drive gear 78, and the second engagement element 52, but is not transmitted to the first engagement element 51. Similarly, the rotation of the first spindle unit SP1 is transmitted to the second driven gear 81, the second drive gear 80, and the fourth engagement element 54, but is not transmitted to the third engagement element 53.

In the electric prosthetic leg 1 configured as described above, similarly to the first embodiment, the knee joint mechanism 30 can be extended and bent via the transmission T that transmits the power of the motor M, and an operation and effect described in the electric prosthetic leg 1 of the first embodiment can be obtained. Further, in the electric prosthetic leg 1 according to the second embodiment, since the actuator 55 is switched using the clutch actuator ACT serving as the power source different from the motor M for extending and bending the knee joint mechanism 30, the actuator 55 can be switched more stably.

The present invention is not limited to the embodiments described above, and modifications, improvements, or the like can be made as appropriate. For example, the embodiments described above disclose the electric prosthetic leg as one embodiment of the joint device of the present invention, whereas the present invention is not limited thereto, and may be applied to an upper limb (arm joint), may be applied to an animal other than a human, or may be applied to a robot.

In the above embodiments, the power of one motor M is transmitted to the first transmission mechanism T1 and the second transmission mechanism T2 via the connection and disconnection mechanism 50, whereas the present invention is not limited thereto, and the intermittent mechanisms may be provided between two power sources and the first transmission mechanism T1 and the second transmission mechanism T2, respectively.

The connection and disconnection mechanism 50 is not limited to the dog clutch, and may be another clutch mechanism such as a friction clutch or a centrifugal clutch, or may be a clutchless mechanism such as a continuous transmission gear ratio switching mechanism.

The present specification describes at least the following matters. Although the corresponding constituent elements or the like in the above-described embodiments are shown in parentheses, the present invention is not limited thereto.
(1) The joint device (electric prosthetic leg 1) includes:
   the first member (the knee lower side member 10);
   the second member (the knee upper side member 20);
   the connecting unit (knee joint mechanism 30) connecting the first member and the second member such that an angle formed by the first member and the second member is variable; and
   the expansion and contraction device (expansion and contraction device 40) capable of varying the angle formed by the first member and the second member by expansion and contraction, in which
   the expansion and contraction device includes:
      the power source (motor M); and
      the power transmission unit (transmission T) configured to transmit power of the power source, and
   the power transmission unit includes:
      the first power transmission path (first transmission mechanism T1) through which the power is transmitted at a first transmission gear ratio; and
      the second power transmission path (second transmission mechanism T2) through which the power is transmitted at a second transmission gear ratio different from the first transmission gear ratio.
   According to (1), the connecting unit can be extended and bent via the power transmission unit that transmits the power of the power source. Further, since the power transmission unit includes the two power transmission paths having different transmission gear ratios, the operation speeds and the generation power can of the extension and bending in the connecting unit can be switched.
(2) The joint device according to (1), in which
   the power source outputs rotary power, and
   the expansion and contraction device includes the motion conversion mechanism (first spindle unit SP1) connected to the power transmission unit so as to transmit power, and configured to convert rotary power output from the power transmission unit into translational motion.
   According to (2), since the motion conversion mechanism that converts the rotational motion into the translational motion is provided, small rotary power can be converted into a large expansion and contraction force.
(3) The joint device according to (1) or (2), in which
   the expansion and contraction device includes:
   the connection and disconnection mechanism (connection and disconnection mechanism 50) interposed between the power source and the power transmission unit, and
   the connection and disconnection mechanism includes:
      the first connection and disconnection mechanism (upper clutch 50U) interposed between the power source and the first power transmission path; and
      the second connection and disconnection mechanism (lower clutch 50D) interposed between the power source or another power source and the second power transmission path.
   According to (3), since the connection and disconnection mechanism interposed between the power source and the power transmission unit includes the first connection and disconnection mechanism interposed between the power source and the first power transmission path and the second connection and disconnection mechanism interposed between the power source or the other power source and the second power transmission path, the two power transmission paths can be appropriately switched.
(4) The joint device according to (3), in which
   the first connection and disconnection mechanism includes the first engagement element (first engagement element 51) that is an engagement element on the power source side and a second engagement element (second engagement element 52) that is an engagement element on the first power transmission path side,
   the second connection and disconnection mechanism includes the third engagement element (third engagement element 53) that is an engagement element on the power source side and the fourth engagement element (fourth engagement element 54) that is an engagement element on the second power transmission path side, and
   the connection and disconnection mechanism further includes the actuator (actuator 55) disposed to be switchable between the first state (first speed change state) in which the first engagement element and the second engagement element are connected to each other and the third engagement element and the fourth engagement element are disconnected from each other, and the second state (second speed change state) in which the first engagement element and the second engagement element are disconnected from each other and the third engagement element and the fourth engagement element are connected to each other.
   According to (4), since the connection and disconnection mechanism further includes the actuator disposed to be switchable between the first state in which the first engagement element and the second engagement element are connected to each other and the third engagement element and the fourth engagement element are disconnected from each other, and the second state in which the first engagement element and the second engagement element are disconnected from each other and the third engagement element and the fourth engagement element are connected to each other, the two power transmission paths can be prevented from being connected at the same time.
(5) The joint device according to (4), in which
   the actuator switches between the first state and the second state with translational motion, and
   the expansion and contraction device further includes the second motion conversion mechanism (second spindle unit SP2) configured to convert the rotary power output from the power source into the translational motion of the actuator.
   According to (5), since the expansion and contraction device further includes the second motion conversion mechanism that converts the rotary power output from the power source into the translational motion of the actuator, the control of the actuator and the control of the extension and the bending in the connecting unit can be implemented by one power source.
(6) The joint device according to (5), in which
   the actuator
   moves to one side in a translation direction of the translational motion when the power source rotates in a first direction, and
   moves to another side in the translation direction when the power source rotates in a second direction opposite to the first direction.
   According to (6), the moving direction of the actuator can be changed by changing the rotation direction of the power source.
(7) The joint device according to (4), in which
   the actuator switches between the first state and the second state with translational motion, and
   the expansion and contraction device further includes:
      the second power source (clutch actuator ACT) different from the power source; and
      the second power transmission unit (clutch fork 90) configured to transmit power of the second power source to the actuator.
   According to (7), since the actuator is switched using the power source different from the power source for extending and bending the connecting unit, the actuator can be switched more stably.
(8) The joint device according to (7), in which
   the actuator includes:
      the intermediate transmission member (slide clutch 56) that is constantly engaged with the second power transmission unit; and
      the bearing (bearing 57) configured to perform the translational motion together with the intermediate transmission member, and
   the bearing includes:
      an outer ring (outer ring 57a) non-rotatably supported by the intermediate transmission member;
      the inner ring (inner ring 57b) configured to rotate integrally with the first engagement element and the third engagement element; and
      the rolling element (rolling element 57c) that is disposed between the outer ring and the inner ring and allows relative rotation between the outer ring and the inner ring.
   According to (8), the first state and the second state can be switched by the translational motion of the intermediate transmission member and the bearing. On the other hand, since the rotation of the first engagement element and the third engagement element is not transmitted to the intermediate transmission member, a degree of freedom of arrangement of the second power transmission unit and the second power source is improved.
(9) The joint device according to any one of (4) to (8), in which
   the actuator is disposed to be switchable between
   the first state,
   the second state, and
   the third state (free state) in which the first engagement element and the second engagement element are disconnected from each other and the third engagement element and the fourth engagement element are disconnected from each other.
   According to (9), in addition to the first state and the second state, the actuator is in the third state in which the power source and the power transmission unit are separated from each other, and thereby the power transmission between the power source and the power transmission unit can be interrupted.
(10) The joint device according to any one of (1) to (9), in which
   the first transmission gear ratio has a speed reduction ratio larger than that of the second transmission gear ratio, and
   the first power transmission path is disposed closer to the power source than the second power transmission path.
   According to (10), since the first power transmission path having a large speed reduction ratio has a small drive gear, the power source can be disposed close to the first power transmission path. Accordingly, even when the drive source is mounted with the joint device as the electric prosthetic leg, an increase in a size of the joint device can be prevented.
(11) The joint device according to (10), in which
   when the connecting unit extends from a bent state, the power of the power source is transmitted via the first power transmission path.
   According to (11), since larger power is required when the knee is extended from the bent state when going up the stairs, when the connecting unit is extended, the power of the power source is transmitted via the first power transmission path having a large speed reduction ratio, and thereby a user can go up the stairs smoothly.
(12) The joint device according to any one of (1) to (11), in which
   the expansion and contraction device further includes a motion conversion mechanism (first spindle unit SP1) that is connected to the power transmission unit so as to transmit power, and configured to convert rotary power output from the power transmission unit into translational motion, and
   when viewed along a rotation axis of the connecting unit, between a narrow angle region in which the formed angle is less than 180° and a wide angle region in which the formed angle is 180° or more when the connecting unit is in a bent state, the motion conversion mechanism is disposed in the narrow angle region.
   According to (12), since the motion conversion mechanism is disposed in the narrow angle region, the bending and extension of the connecting unit can be implemented by the translational motion of the motion conversion mechanism.
(13) The joint device according to (12), in which
   the power source is disposed in the wide angle region.
   According to (13), by disposing the motion conversion mechanism in the narrow angle region and disposing the power source in the wide angle region, the motion conversion mechanism and the power source can be disposed in a well-balanced manner with the rotating portions of the connecting unit sandwiched therebetween.
(14) The joint device according to (13), in which
   the power transmission unit is disposed between the power source and the motion conversion mechanism.
   According to (14), by disposing the power transmission unit between the power source and the motion conversion mechanism, the power source, the power transmission unit, and the motion conversion mechanism can be collectively disposed.
(15) The joint device according to (14), in which
   the expansion and contraction device further includes an connection and disconnection mechanism (connection and disconnection mechanism 50) interposed between the power source and the power transmission unit, and
   the connection and disconnection mechanism is disposed between the power source and the motion conversion mechanism.
   According to (15), by disposing the connection and disconnection mechanism between the power source and the motion conversion mechanism, the power source, the power transmission unit, the connection and disconnection mechanism, and the motion conversion mechanism can be collectively disposed.
(16) The joint device according to any one of (1) to (15), in which
   the joint device is an electric prosthetic leg.

According to (16), the required operation speeds and the generation power are different when the knee is bent and extended when going up the stairs, and thus the power transmission paths can be changed when the knee is bent and extended.

The present application is based on a Japanese Patent Application (Japanese Patent Application No. 2019-157358) filed on August 29, 2019, the contents of which are incorporated herein by reference.

### REFERENCE SIGNS LIST

1 electric prosthetic leg (joint device)
10 knee lower side member (first member)
20 knee upper side member (second member)
30 knee joint mechanism (connecting unit)
40 expansion and contraction device
50D lower clutch (second connection and disconnection mechanism)
50U upper clutch (first connection and disconnection mechanism)
50 connection and disconnection mechanism
51 first engagement element
52 second engagement element
53 third engagement element
54 fourth engagement element
55 actuator
56 slide clutch (intermediate transmission member)
57 bearing
57a outer ring
57b inner ring
57c rolling element
90 clutch fork (second power transmission unit)
ACT clutch actuator (second power source)
M motor (power source)
T transmission (power transmission unit)
T1 first transmission mechanism (first power transmission path)
T2 second transmission mechanism (second power transmission path)
SP1 first spindle unit (motion conversion mechanism)
SP2 second spindle unit (another motion conversion mechanism)

## Claims

1. A joint device comprising:
a first member;
a second member;
a connecting unit connecting the first member and the second member such that an angle formed by the first member and the second member is variable; and
an expansion and contraction device capable of varying the angle formed by the first member and the second member by expansion and contraction, wherein
the expansion and contraction device includes:
a power source; and
a power transmission unit configured to transmit power of the power source, and
the power transmission unit includes:
a first power transmission path through which the power is transmitted at a first transmission gear ratio; and
a second power transmission path through which the power is transmitted at a second transmission gear ratio different from the first transmission gear ratio.

2. The joint device according to claim 1, wherein
the power source outputs rotary power, and
the expansion and contraction device further includes a motion conversion mechanism connected to the power transmission unit so as to transmit power, and configured to convert rotary power output from the power transmission unit into translational motion.

3. The joint device according to claim 1 or 2, wherein
the expansion and contraction device further includes an connection and disconnection mechanism interposed between the power source and the power transmission unit, and
the connection and disconnection mechanism includes:
a first connection and disconnection mechanism interposed between the power source and the first power transmission path; and
a second connection and disconnection mechanism interposed between the power source or another power source and the second power transmission path.

4. The joint device according to claim 3, wherein
the first connection and disconnection mechanism includes a first engagement element that is an engagement element on the power source side and a second engagement element that is an engagement element on the first power transmission path side,
the second connection and disconnection mechanism includes a third engagement element that is an engagement element on the power source side and a fourth engagement element that is an engagement element on the second power transmission path side, and
the connection and disconnection mechanism further includes an actuator disposed to be switchable between a first state in which the first engagement element and the second engagement element are connected to each other and the third engagement element and the fourth engagement element are disconnected from each other, and a second state in which the first engagement element and the second engagement element are disconnected from each other and the third engagement element and the fourth engagement element are connected to each other.

5. The joint device according to claim 4, wherein
the actuator switches between the first state and the second state with translational motion, and
the expansion and contraction device further includes a second motion conversion mechanism configured to convert rotary power output from the power source into the translational motion of the actuator.

6. The joint device according to claim 5, wherein
the actuator
moves to one side in a translation direction of the translational motion when the power source rotates in a first direction, and
moves to another side in the translation direction when the power source rotates in a second direction opposite to the first direction.

7. The joint device according to claim 4, wherein
the actuator switches between the first state and the second state with translational motion, and
the expansion and contraction device further includes:
a second power source different from the power source; and
a second power transmission unit configured to transmit power of the second power source to the actuator.

8. The joint device according to claim 7, wherein
the actuator includes:
an intermediate transmission member that is constantly engaged with the second power transmission unit; and
a bearing configured to perform the translational motion together with the intermediate transmission member, and
the bearing includes:
an outer ring non-rotatably supported by the intermediate transmission member;
an inner ring configured to rotate integrally with the first engagement element and the third engagement element; and
a rolling element that is disposed between the outer ring and the inner ring and allows relative rotation between the outer ring and the inner ring.

9. The joint device according to any one of claims 4 to 8, wherein
the actuator is disposed to be switchable between
the first state,
the second state, and
a third state in which the first engagement element and the second engagement element are disconnected from each other and the third engagement element and the fourth engagement element are disconnected from each other.

10. The joint device according to any one of claims 1 to 9, wherein
the first transmission gear ratio has a speed reduction ratio larger than that of the second transmission gear ratio, and
the first power transmission path is disposed closer to the power source than the second power transmission path.

11. The joint device according to claim 10, wherein
when the connecting unit extends from a bent state, the power of the power source is transmitted via the first power transmission path.

12. The joint device according to any one of claims 1 to 11, wherein
the expansion and contraction device further includes a motion conversion mechanism that is connected to the power transmission unit so as to transmit power, and configured to convert rotary power output from the power transmission unit into translational motion, and
when viewed along a rotation axis of the connecting unit, between a narrow angle region in which the formed angle is less than 180° and a wide angle region in which the formed angle is 180° or more when the connecting unit is in a bent state, the motion conversion mechanism is disposed in the narrow angle region.

13. The joint device according to claim 12, wherein
the power source is disposed in the wide angle region.

14. The joint device according to claim 13, wherein
the power transmission unit is disposed between the power source and the motion conversion mechanism.

15. The joint device according to claim 14, wherein
the expansion and contraction device further includes an connection and disconnection mechanism interposed between the power source and the power transmission unit, and
the connection and disconnection mechanism is disposed between the power source and the motion conversion mechanism.

16. The joint device according to any one of claims 1 to 15, wherein
the joint device is an electric prosthetic leg.
